# EUROPÄISCHE PATENTANMELDUNG

(11) **EP 3 417 870 A1**
(43) Veröffentlichungstag der Anmeldung: **26.12.2018**
(21) Anmeldenummer: 18179219.3
(22) Anmeldetag: 22.06.2018
(51) Int. Cl.: A61K 36/899

(54) **ORAL EINZUNEHMENDE ZUBEREITUNGEN MIT ANTHOCYANINEN AUS BLAUEN MAIS-SPINDELN**

(30) Priorität: 22.06.2017 CH 8232017
(71) Anmelder: Acopan AG, 9524 Zuzwil (CH)
(72) Erfinder: WOLFGANG ENGEL, Dieter, 9524 Zuzwil (CH); ROBERT BROCKER, Erich, 9533 Kirchberg (CH)
(74) Vertreter: Hepp Wenger Ryffel AG

(57) **Zusammenfassung**

Die vorliegende Erfindung, Zubereitung, enthaltend einen Anthocyanin-haltigen Extrakt aus den Spindeln und/oder den äusseren Hüllblättern von blauem Mais, vorzugsweise Mais Morado, zur oralen Behandlung von und/oder Vorbeugung gegen chronisch-entzündliche Darmerkrankungen beim Menschen, wobei die chronisch-entzündliche Darmerkrankung vorzugsweise ausgewählt ist aus der Gruppe bestehend aus Colitis, insbesondere von Colitis Irritabile, Colitis Ulcerosa, Morbus Crohn, und Reizdarm-Syndrom.

## Beschreibung

Anthocyaninreiche Spezies von Mais (Zea mays L.), beispielsweise Mais Morado (Purple Corn), sind seit längerem, insbesondere in Peru und Mexico, bekannt und mögen möglicherweise zurückgehen bis auf die Zeit vor der Conquista. Bestimmte Phänotypen mit sehr hohem Gehalt an Anthocyaninen sind zum Beispiel aus den Patenten CN 1545854 oder US-8,124,143 B2, US-8,153,866 B2, US,8,491,670 B2 oder US-8,642,847 B2 und der Literatur (Lopez-Martinez, Oliart-Ros et al., LWT-Food science and Technology 42(6): 1187-1192 (2009)) bekannt. Ausserdem ist deren Gehalt an spezifischen Anthocyaninen- insbesondere Cyanidin-3-glucosid - bekannt. Verschiedene Kultivare und verschiedene Pflanzenteile wurden bezüglich Anthocyaninen charakterisiert.

Eine Methode der wässrigen Extraktion von Anthocyaninen aus der Oberfläche (Epicarp, Mesocarp) von Mais morado-Körnern zur kommerziellen Farbstoffgewinnung ist bekannt (z.B. US-8,124,143 B2, US-8,153,866 B2, US,8,491,670 B2 oder US-8,642,847 B2). Weiterhin ist die Extraktherstellung aus Mais morado-Spindeln oder Maiskolbenhüllblättern (husk) aus der Literatur bekannt (De Pascual-Teresa, Santos-Buelga et al., Journal of the Science of Food and Agriculture 82(9): 1003-1006 (2002), Li, Kim et al., J Agric. Food Chem 56(23): 11413-11416 (2008)).

Die Verwendung der Anthocyanin-Extrakte aus Mais Morado (Purple Corn) zur Färbung (Jing und Giusti, J Agric Food Chem 53(22): 8775-8781 (2005)) oder zu Gesundheitszwecken(WO-2007/038421; KR-2015/0125339; Jin, PURPLE CORN ANTHOCYANINS: CHEMICAL STRUCTURE, CHEMOPROTECTIVE ACTIVITY AND STRUCTURE/FUNCTION RELATIONSHIPS, Dissertation, Ohio State University (2006); Lao, Sigurdson et al., Comprehensive Reviews in Food Science and Food Safety 16: 234-246 (2017); Yang und Zhai, Innovative Food Science & Emerging Technologies 11(1): 169-176 (2010)) wurde beschrieben. Dabei werden allgemeine Anwendungen ohne klinische Symptome (wie zum Beispiel antioxidative oder chemopräventive Wirkung) oder Krankheiten wie Diabetes, Darmkrebs (Hagiwara, Miyashita et al., Cancer Lett. 171(1): 17-25 (2001)) oder Prostatakrebs (Long, Suzuki et al., Cancer Sci 104(3): 298-303 (2013)) genannt. Dabei wurden nicht nur die bekannten Anthocyanine und deren Aglycone, sondern auch weitere enthaltene Polyphenole in Purple Corn durch selektive/mehrfache Extraktion erhalten und geprüft. In keiner Literatur wird allerdings die vorteilhafte Anwendung der Purple Corn Extrakte als solche gegen entzündliche Darmerkrankungen aufgeführt.

Gesundheitliche Wirkungen einzelner Anthocyanine, die auch in Purple Corn gefunden werden können, sind zum Teil ebenfalls bekannt: Chen, Chu et al. (Nutr. Cancer 53(2): 232-243 (2005)) beschreibt die Wirkungen von Cyanidin-3-glucosid und Peonidin-3-glucosid aus Oriza Sativa; Kwon, Lee et al. (Ann N Y Acad Sci 1095: 513-520, (2007)) solche von Peonidin.

Der nach oraler Gabe von Anthocyaninen zu erwartende Abbau im Magendarmtrakt und die Wechselwirkung zwischen Darmflora und Nahrungsbestandteilen wurde in Teilen durch Kamiloglu, Capanoglu et al. (Int J Mol Sci 16(9): 21555-21574 (2015)) und Kahkonen und Heinonen (J Agric Food Chem 51(3): 628-633 (2003)) exploriert.

Einen Einfluss von Anthocyaninen auf experimentell induzierte Colitis in Ratten ist von Jansakova, Babickova et al. (Folia Biol (Praha) 61(3): 104-109 (2015)) publiziert worden. Die Identität von Anthocyaninen aus *"anthocyanin-rich wheat food"* ist jedoch mehr als unklar. Weiterhin geben EP-2 135 616 A1 und Piberger, Oehme et al. (Mol Nutr Food Res 55(11): 1724-1729 (2011)) sowie Montrose, Horelik et al. (Carcinogenesis 32(3): 343-350 (2011)) Hinweise zur Wirkung von Anthocyaninen aus "Bilberry" aus "Black Raspberries" auf entzündliche Darmerkrankungen. Ein neuerer Übersichtsartikel von Vezza, Rodriguez-Nogales et al. (Nutrients 8(4): 211 (2016)) über entzündliche Darmerkrankungen diskutiert verschiedenste Faktoren, die zu einer entzündlichen Darmerkrankung beitragen können, gibt aber keine Hinweise auf Extrakte/Anthocyanine aus Purple Corn.

EP-1 718 312 B1 beansprucht die Verwendung von Hopfenextrakt mit hohem Anteil an Alphasäuren für entzündliche Darmerkrankungen.

Aus Naturprodukten erhältliche Extrakte sind von heterogenem Aufbau und können abhängig vom Naturprodukt und der gewählten Extraktionsmethode in ihrer Zusammensetzung variieren. Die in einem Extrakt enthaltenen Inhaltsstoffe können aufeinander synergistische oder auch antagonistische Effekte ausüben. Es können daher von einem Naturprodukt-Extrakt nur sehr bedingt Rückschlüsse auf einen anderen Naturstoff-Extrakt gezogen werden.

Unter dem Sammelbegriff *"chronisch-entzündliche Darmerkrankungen"* (CED) werden Krankheitsbilder zusammengefasst, die sich durch schubweise rezidivierende oder kontinuierlich auftretende, entzündliche Veränderungen des Darms auszeichnen. Die wichtigsten chronisch-entzündlichen Darmerkrankungen sind Morbus Crohn (MC) und Colitis Ulcerosa (CU). Darüber hinaus beinhaltet der Begriff CED seltenere entzündliche Darmerkrankungen wie die Collagene Colitis (CC)oder lymphozytäre Colitis.

Als Vorstufe zu den CED kann das sogenannte Reizdarmsyndrom (irritable bowel syndrome (IBS) oder auch Colon Irritabile (Reizkolon) genannt) gezählt werden, da gewisse typische Gewebeveränderungen wie bei CED festgestellt werden können.

Obwohl für Patienten mit ulcerativer Colitis ein gewisses Risiko besteht Darmkrebs zu entwickeln, sind klinisches Bild und Therapie von Darmkrebs und entzündlichen Darmerkrankungen komplett verschieden.

Entzündliche Darmerkrankungen sind verbreitet (bis zu 20% der Bevölkerung), aber über deren Entstehung sind wenige gesicherte Ursache-Wirkungsbeziehungen bekannt. Insbesondere die IBS ist zusätzlich stark von psychischen Faktoren geprägt.

Neben den vielen bekannten, zum Teil individuell wirkenden Auslösern der Entzündungen in der Nahrung können aber auch Faktoren wie die Zusammensetzung der Darmflora, prä- und probiotische Anteile der Nahrung, sowie weitere sekundäre Pflanzeninhaltsstoffe einen positiv mildernden Einfluss auf die Entzündungshäufigkeit und -schwere haben. Dabei sind mehrere Faktoren der mechanistischen Wirkung wie antioxidative Wirkung, Entzündungshemmung, Einfluss auf die Darmflora, immun-modulierender Effekt etc. - je nach Stoff - festzustellen (Farzaei, Bahramsoltani et al. Expert Rev Gastroenterol Hepatol: 1-14 (2016)), so dass die bisher den Anthocyaninen zugeschriebenen Wirkungen in verschiedenen Testsystemen mit Vorbehalten beurteilt werden müssen.

Neuere Forschungsresultate zeigen (Vezza, Rodriguez-Nogales et al., Nutrients 8(4): 211 (2016)), dass insbesondere Anthocyanine aus Heidelbeere, oder Himbeeren einen positiven Einfluss auf die Abheilung oder Linderung entzündlicher Darmerkrankungen haben können. Wirksam scheinen die Anthocyanine selbst und weniger die Aglycone zu sein. Auch tritt die Wirkung offenbar weniger systemisch als vor allem lokal im Darm nach oraler Aufnahme und unter Modulation der vorhandenen Darmflora ein. Um die Wirkung zu erreichen, sind aber bisher sehr hohe Einnahmemengen vorzusehen, worunter die Compliance der Patienten leidet.

Die hohe erforderliche Dosis könnte durch die Spaltung der selektiv wirkenden Glycoside in ihre weniger wirksamen Aglycone durch das Enzymsystem des Patienten oder der Darmflora bedingt sein.

Es bestand somit die Aufgabe der vorliegenden Erfindung, eine Zubereitung gegen chronisch-entzündliche Darmerkrankungen (CED) bereitzustellen, die insbesondere in akzeptabler (d.h. nicht zu grosser) Einnahmemenge und zu ökonomisch erschwinglichen Kosten in dafür geeignete Darreichungsformen verabreicht werden kann und gute Wirksamkeit gegen diese entzündlichen Darmerkrankungen hat.

Überraschend hat sich gezeigt, dass gegen CED wirksame Zubereitungen auch aus besonderen Pflanzen bzw. deren Pflanzenteilen angereichert werden können, die leicht und kostengünstig angebaut werden können. Die vorliegende Erfindung betrifft daher eine Zubereitung, enthaltend einen Anthocyanin-haltigen Extrakt aus den Spindeln und/oder den äusseren Hüllblättern von blauem Mais; (Mais Morado; purple Corn; Zea mays L.) zur oralen Behandlung und/oder Vorbeugung gegen chronisch-entzündliche Darmerkrankungen beim Menschen.

Gemäss der vorliegenden Erfindung werden die Begriffe "Zubereitung" und "Zubereitungen" synonym verwendet und betreffen sowohl eine einzige als auch mehrere erfindungsgemässe Zubereitungen.

Gemäss der vorliegenden Erfindung ist der Begriff "und/oder" dahingehend zu verstehen, dass eine der genannten Komponenten allein oder beide Komponenten gemeinsam vorhanden sind.

Gemäss der vorliegenden Erfindung werden unter "Anthocyaninen" (oder der synonymen Bezeichnung "Anthocyane") pflanzliche Inhaltsstoffe verstanden, welche ein Benzopyrylium-Ion als charakteristisches Strukturmerkmal enthalten. Anthocyanine bestehen aus einem sogenannten Aglycon-Teil, welcher als "Anthocyanidin" bezeichnet wird, und einem Zuckerrest (Saccharidrest oder Glucosidrest).

Die am häufigsten natürlich vorkommenden Anthocyanine sind durch folgende Anthocyanidin-Strukturen (d.h. die entsprechenden Aglyconteile) charakterisiert:

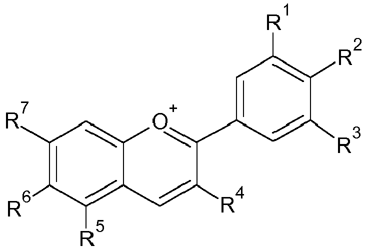

| Anthocyanin | R1 | R2 | R3 | R4 | R5 | R6 | R7 |
|---|---|---|---|---|---|---|---|
| Aurantinidin | H | OH | H | OH | OH | OH | OH |
| Cyanidin | OH | OH | H | OH | OH | H | OH |
| Delphinidin | OH | OH | OH | OH | OH | H | OH |
| Europinidin | OCH₃ | OH | OH | OH | OCH₃ | H | OH |
| Luteolinidin | OH | OH | H | H | OH | H | OH |
| Malvidin | OCH₃ | OH | OCH₃ | OH | OH | H | OH |
| Pelargonidin | H | OH | H | OH | OH | H | OH |
| Peonidin | OCH₃ | OH | H | OH | OH | H | OH |
| Petunidin | OH | OH | OCH₃ | OH | OH | H | OH |
| Rosinidin | OCH₃ | OH | H | OH | OH | H | OCH₃ |

Der Zuckerrest ist bei den Anthocyaninen über eine glykosidische Bindung an das Anthocyanidin (Aglyconteil) gebunden. Es können auch mehr als ein Zuckerrest an den Aglyconteil gebunden sein. In natürlich vorkommenden Anthocyaninen sind die Zuckerreste üblicherweise ausgewählt aus Glucosid-, Galactosid-, Rutinosid- oder Sambubibosidresten. Beispielhaft sei die Struktur von Cyanidin-3-O-glucosid gezeigt:

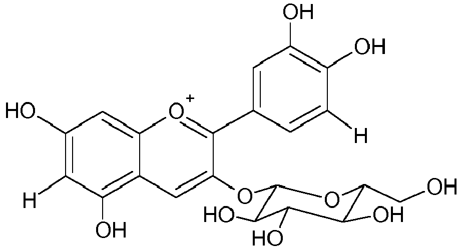

Natürlich vorkommende Polyphenole sind eine Gruppe phytochemischer Substanzen mit heterogener Struktur. Natürlich vorkommende Polyphenole werden üblicherweise unterteilt in hydrolysierbare Tannine (Gallsäureester von Glucose und anderen Zuckern) und Phenylpropanoide, wie Lignine, Flavonoide und kondensierte Tannine. Beispielhaft sei nachstehend Hirsutrin gezeigt:

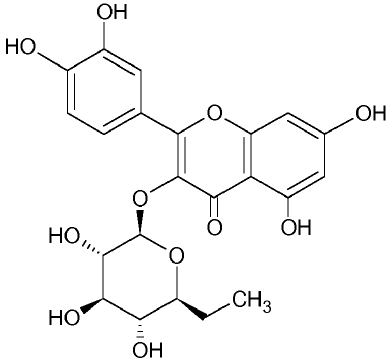

Erfindungsgemäss können Zubereitungen zur Verabreichung der notwendigen Mengen der wirksamen Inhaltsstoffe in von Patienten bevorzugten Darreichungsformen bei einer hohen Bioverfügbarkeit am Ort der gewünschten Wirkung (z.B. im Colon) bereitgestellt werden.

Bevorzugte Ausführungsformen der vorliegenden Erfindung sind:
- Zubereitungen, enthaltend einen Anthocyanin-haltigen Extrakt aus den Spindeln von blauem Mais (Mais Morado; purple Corn) (Zea mays L.) zur oralen Behandlung und/oder Vorbeugung gegen chronisch-entzündliche Darmerkrankungen des Menschen.
- Zubereitungen wie vorstehend definiert zur Behandlung und Vorbeugung von Colitis, insbesondere von Colitis Irritabile, Colitis Ulcerosa.
- Zubereitungen wie vorstehend definiert zur Behandlung von Morbus Crohn.
- Zubereitungen wie vorstehend definiert zur Behandlung von Reizdarm Syndrom.
- Zubereitungen wie vorstehend definiert, enthaltend einen anthocyaninhaltigen Trocken-Extrakt, der mit wässrig-alkoholischen, bevorzugt Wasser-Ethanol-Gemischen als Auszugsmittel, bei pH zwischen 2 - 5, bevorzugter zwischen pH 2-3 unter Zusatz von Ascorbinsäure und/oder Betain gewonnen wurde.
- Zubereitungen wie vorstehend definiert, wobei der verwendete Trockenextrakt einen Anthocyanin-Anteil von mindestens 10 %w/w, bevorzugter von 10-40 %w/w noch bevorzugter von 40-70 %w/w aufweist.
- Zubereitungen wie vorstehend definiert in einer festen, halbfesten oder flüssigen Form zur oralen Einnahme.
- Zubereitung wie vorstehend definiert, wobei die Zubereitung ein Pulver, ein Granulat, eine Tablette, eine Hartkapsel, eine Weichkapsel, ein Sirup, ein Dicksaft, ein Gel, eine Fruchtschnitte oder Riegel, ein stabilisiertes oder sterilisiertes Getränk ist.
- Zubereitung wie vorstehend definiert, wobei die Zubereitung in Form eines Arzneimittels, eines Medizinproduktes, eines Nahrungsergänzungsmittels, als Lebensmittel oder in einer einem Genussmittel ähnlichen Form angeboten wird.
- Zubereitungen wie vorstehend definiert, dadurch gekennzeichnet, dass die Zubereitungen aus Purple Corn mit Extrakten reich an Alpha-Säuren von Hopfen (Humulus Lupulus) kombiniert werden.

Gemäss der vorliegenden Erfindung werden die beanspruchten Zubereitungen aus den Spindeln und/oder den äusseren Hüllblättern des Maiskolbens von Mais, insbesondere blauem Mais (Purple Corn, Zea Mays L.) gewonnen, einer schon seit mehreren Jahrhunderten in Peru (aber auch Spanien) verbreiteten Maisvarietät.

Da die Spindeln betreffend Energiegehalt und anderweitiger Verwendung praktisch "wertlos" sind, die Konzentration an Inhaltsstoffen wie Anthocyaninen aber vergleichsweise hoch ist und so der Extrakt zu niedrigen Kosten hergestellt werden kann, weisen die erfindungsgemässen Zubereitungen auch ökologische und ökonomische Vorteile auf. Die Extraktion aus Maiskörnern (kernels) ist demgegenüber eher unwirtschaftlich, da die Anthocyanine im Wesentlichen nur in der äusseren Schicht vorhanden sind - im stärkehaltigen Rest sind nur noch wenige Anthocyanine - und zudem Maiskörner als Nahrungsmittel zum Einsatz kommen und entsprechend teurer sind.

Mais, insbesondere Blauer Mais (Mais Morado (Purple Corn, Zea Mays L.)) und sein Anbau sind hinlänglich bekannt und müssen hier nicht näher beschrieben werden.

Ohne eine erschöpfende Theorie den Wirkmechanismus der erfindungsgemässen Zubereitung aus einem Extrakt aus den Spindeln und/oder den äusseren Hüllblättern des Maiskolbens von Purple Corn angeben zu wollen, hat sich überraschenderweise gezeigt, dass Monoglycoside von Anthocyanidinen und gewisse Polyphenole wie Hirsutrin, welche in dem erfindungsgemässen Extrakt in besonderem Masse vorhanden sind, besonders geeignet sind, ohne Spaltung in ihre Aglycone durch Enzyme des Verdauungsapparates des Menschen und die Darmflora bis an den Zielort der unteren Darmsegmente, insbesondere Ileum, Caecum und Colon geführt werden können. Die Mehrzahl der Anthocyanine wird bereits in hohem Mass im Bereich Jejunum zu Aglyconen deacyliert. Es ist bekannt, dass die Aglycone weniger biologische Aktivität als die entsprechenden Anthocyanine für die Behandlung entzündlicher Darmerkrankungen aufweisen.

Die erfindungsgemässen Zubereitungen zeichnen sich dadurch aus, dass die Nachteile eines die Wirkung beeinträchtigenden Abbaus der Inhaltsstoffe durch die Darmflora und/oder das Enzymsystem des Patienten bis an den Zielort vermieden werden.

Gemäss einer bevorzugten Ausführungsform der vorliegenden Erfindung zeichnet sich die erfindungsgemässe Zubereitung durch einen Anteil von Anthocyaninen im Extrakt aus, der im Bereich von mehr als 10 Gew.-%, beispielsweise 10-40 Gew.-%, vorzugsweise 50 bis 80 Gew.-%, bevorzugter 60 bis 75 Gew.-% und besonders bevorzugt 65 bis 75 Gew.-% liegt, bezogen auf die Gesamttrockenmenge des Extrakts.

Gemäss einer besonders bevorzugten Ausführungsform der vorliegenden Erfindung zeichnet sich die erfindungsgemässe Zubereitung durch einen Anteil an Anthocyaninen in Form von Monoglycosiden aus, der im Bereich von 30 bis 90 Gew.-%, bevorzugt 50 bis 80 Gew.-%, vorzugsweise 60 bis 75 Gew.-% und besonders bevorzugt 60 bis 70 Gew.-% liegt, bezogen auf die Gesamttrockenmenge der enthaltenen Anthocyanine.
Gemäss einer bevorzugten Ausführungsform der vorliegenden Erfindung zeichnet sich die erfindungsgemässe Zubereitung durch einen zusätzlichen Anteil von Polyphenolen im Extrakt aus, der im Bereich von 1 bis 10 Gew.-%, vorzugsweise 2 bis 8 Gew.-% und besonders bevorzugt 3 bis 6 Gew.-% liegt, bezogen auf die Gesamttrockenmenge des Extrakts.

Gemäss einer weiteren bevorzugten Ausführungsform der vorliegenden Erfindung zeichnet sich die erfindungsgemässe Zubereitung durch einen Anteil von bis zu 20 Gew.-% Phenylpropanoid-Sucrosiden (zum Beispiel Mono, Di-, Tri- und Tetracetyldiferuloylsucrose) aus.

Erfindungsgemäss wird unter Gesamttrockenmenge die gesamte Menge der entsprechenden Komponente im getrockneten Zustand verstanden, d.h. nach Entfernung von flüchtigen Lösungsmitteln, welche während der Extraktion eingesetzt werden.

Die Auswahl von genetisch gut charakterisiertem Pflanzengut mit hohem Anteil an Anthocyaninen in der Spindel, kombiniert mit hoher Konzentration an den gewünschten spezifischen Inhaltsstoffen wie Anthocyaninen, kombiniert mit optimal auf die Extraktion ausgerichteten Bedingungen (Ausbeute, Zerstörungsfreiheit, Anreicherung der richtigen Molekülformen der Anthocyanine (bzw. anderen Gluosiden) ist zielführend für eine wirksamen und ökonomisch optimierte erfindungsgemässe Zubereitung.

Ein weiterer erfindungsgemässer Aspekt betrifft die Herstellung eines anthocyaninreichen Extraktes für die erfindungsgemässe Zubereitung.

Um die erfindungsgemässen Zubereitungen enthaltend Anthocyanine als eine Wirkstoffklasse aus einer Maisspindel und/oder den äusseren Hüllblättern ohne Zerstörung/Degradation dieser Inhaltsstoffe zu Aglyconen zu extrahieren, oder ohne entsprechende Kondensationsreaktionen zu polymeren Anthocyaninen zu provozieren, sind bestimmte erfindungsgemässe Vorbehandlungs- und Extraktionsmethoden notwendig.

Die vorliegende Erfindung betrifft ein Verfahren zur Herstellung einer vorstehend beschriebenen Zubereitung, umfassend den Schritt der Behandlung der Spindel und/oder der äusseren Hüllblättern von blauem Mais, vorzugsweise Mais Morado, mit einem wässrigen Medium, vorzugsweise einem wässrig-alkoholischen Medium, besonders bevorzugt einem Wasser-Ethanol-Gemisch, wobei das wässrige Medium einen pH-Wert von 2 bis 5, bevorzugt 2 bis 3 aufweist.

Erfindungsgemäss bevorzugt werden die Spindel und/oder der äusseren Hüllblättern von blauem Mais, vorzugsweise Mais Morado, vor der Behandlung mit wässrigem Medium zerkleinert, um den Extraktionsschritt zu fördern. Vorzugsweise erfolgt eine Zerkleinerung zu Partikeln mit einer durchschnittlichen Partikelgrösse von 1- 10 mm, bevorzugt 1-5 mm. Die Zerkleinerung kann mit üblichen Zerkleinerungsgeräten durchgeführt werden, beispielsweise mit einer Hammermühle.

Gemäss einer weiteren bevorzugten Ausführungsform können die so erhaltenen Partikel vor der eigentlichen Extraktion einer Mazeration und/oder Perkolation unterworfen werden, d.h. einem Einweichen oder Durchsickern (beispielsweise durch Gabe der Partikel auf einen Filter und Übergiessen) mit einem geeigneten Lösungsmittel oder Lösungsmittelgemisch. Erfindungsgemäss bevorzugt wird hierbei das gleiche wässrige Medium verwendet wie bei der anschliessenden Extraktion.

Erfindungsgemäss bevorzugt ist das bei der Extraktion verwendete wässrige Medium ein Wasser-Ethanol-Gemisch, wie vorstehend beschrieben. Besonders bevorzugt ist hierbei ein Wasser-Ethanol-Gemisch mit einem Verhältnis von Wasser:Ethanol von 90:10 bis 60:40, insbesondere bevorzugt 85:30 und speziell 80:20.

Erfindungsgemäss erfolgt die Extraktion unter leicht sauren Bedingungen, bei einem pH-Wert von 2 bis 5, bevorzugt 2 bis 3. Die sauren Bedingungen können auf übliche Weise durch Zugabe einer herkömmlichen Säure, beispielsweise verdünnte (z.B. 0.1%) Salzsäure, eingestellt werden.

Bei der Extraktion können folgende Zusätze die Extraktausbeute bzw. die Löslichkeit und damit die Bioverfügbarkeit (lokal im Colon) des Trockenextraktes erhöhen: Mineralsäuren (pH-Absenkung), Organische Säuren (pH-Absenkung, organische Gegenionen verbessern die Eigenschaften und Lösefähigkeit des einmal getrockneten Extraktes), Ascorbinsäure (Reduzierendes Medium während der Extraktion), MCC (Mikrokristalline Cellulose; Stellmittel, Anticaking), Magnesiumsalze/Magnesiumsilikate (Anticaking, Dispergierhilfen im getrockneten Extrakt), Betain (als Zwitterion, Erhöhung der Löslichkeit, Koordination der Anthocyanine, Anti-inflammatorische Komponente). Es hat sich erfindungsgemäss als besonders bevorzugt erwiesen, die Extraktion unter Zusatz von Ascorbinsäure und/oder Betain durchzuführen. Diese Stoffe werden in für den Fachmann üblichen Mengen zugegeben, welche 5% des Gewichts der zu extrahierenden Partikel üblicherweise nicht überschreiten.

Die Extraktion erfolgt erfindungsgemäss unter üblichen Bedingungen einer derartigen Extraktion, d.h. in einer geeigneten Apparatur wie einem Soxhlet-Extraktor unter erhöhter Temperatur über einen ausreichenden Zeitraum. Erfindungsgemäss bevorzugt wird die Extraktion bei einer Temperatur von 30 bis 90°C, vorzugsweise 40 bis 70°C, über einen Zeitraum von 1 bis 24 h, beispielsweise 5 bis 15 h durchgeführt.

Die nach der Extraktion erhaltene flüssige Phase kann auf übliche Weise gereinigt werden (beispielsweise durch Filtration) und wird anschliessend durch Entfernung des Lösungsmittels auf übliche Weise (ggfs. unter Zugabe eines Trägerstoffes wie mikrokristalliner Cellulose) in einen Trocken-Extrakt überführt. Dieser kann wie erhalten in der erfindungsgemässen Zubereitung eingesetzt oder bei Bedarf auf eine gewünschte Partikelgrösse zerkleinert werden. Die Zerkleinerung kann mit üblichen Zerkleinerungsgeräten durchgeführt werden, beispielsweise mit einer Hammermühle. Eine erfindungsgemäss bevorzugte Partikelgrösse des Trocken-Extrakts beträgt 50-300 µm, vorzugsweise 100-200 µm.

Bedingt durch das wässrige Auszugsmittel wird im Extrakt ein relativ hoher Anteil an Sacchariden/Polyschariden, Proteinen und Salzen sowie weiteren wasserlöslichen sekundären Pflanzeninhaltsstoffen gefunden. Andererseits resultiert durch die Verwendung von Maisspindeln, die im Wesentlichen aus wasserunlöslichen Polysacchariden (Lignin, Cellulose) bestehen, ein "Drogen:Extrakt-Verhältnis" von 25-50:1; d.h. die Ausbeute an löslichen Bestandteilen ist relativ gering. Dies unterstreicht die Notwendigkeit eines ökonomischen, kostengünstigen Ausgangsmaterials. Maisspindeln sind in dieser Hinsicht erfindungsgemäss besonders bevorzugt.

Gemäss einer bevorzugten erfindungsgemässen Ausführungsform ist die Anreicherung bestimmter, besonders wirksamer Inhaltsstoffe wie Anthocyanine gegen CED, bei der Gewinnung der erfindungsgemässen Zubereitung durch Extraktion und Aufreinigung besonders zielführend. Dies wird durch das vorstehend beschriebene erfindungsgemässe Herstellungsverfahren erreicht.

Die erfindungsgemässe Zubereitung enthält den Anthocyaninhaltigen Extrakt aus den Spindeln und/oder den äusseren Hüllblättern von blauem Mais, vorzugsweise Mais Morado, vorzugsweise in einer Menge von 50 bis 90 Gew.-%, bevorzugter 60 bis 80 Gew.-%, bezogen auf das Gesamtgewicht der Zubereitung.

Die erfindungsgemässe Zubereitung kann in jeder üblichen, für die orale Gabe geeigneten Darreichungsform bereitgestellt werden. Geeignete feste, halbfeste oder flüssige galenische Zubereitungsformen sind beispielsweise ein Granulat, Pulver, Dragees, Tabletten, (Mikro)Kapseln, Sirup/Dicksaft, Suspensionen, Emulsionen, Tropfen, ein Gel, eine Fruchtschnitte oder Riegel, oder ein stabilisiertes oder sterilisiertes Getränk.

Bei der Herstellung dieser Darreichungsformen finden übliche Hilfsmittel, wie Trägerstoffe, Spreng-, Binde-, Überzugs-, Quellungs-, Gleit- oder Schmiermittel, Geschmacksstoffe, Süßungsmittel oder Lösungsvermittler, Verwendung. Als häufig verwendete Hilfsstoffe seien z. B. Magnesiumcarbonat, Titandioxid, Laktose, Mannit und andere Zucker, Talkum, Milcheiweiß, Gelatine, Stärke, Cellulose und ihre Derivate, tierische und pflanzliche Öle, Polyethylenglykole und Lösungsmittel, wie etwa steriles Wasser und ein- oder mehrwertige Alkohole, z.B. Glycerin, genannt.

Diese Hilfsstoffe werden in für die entsprechenden Darreichungsformen üblichen Mengen zugesetzt, beispielsweise in einer Menge von jeweils 0.1 bis 10 Gew.-%, bezogen auf das Gesamtgewicht der Zubereitung.

Der Zusatz der Hilfsstoffe zum Extrakt bzw. zu den Zubereitungsformen ist notwendig, um die hohe Konzentration an bestimmten Anthocyaninen chemisch über die Lager- und Anwendungszeit stabil zu halten, sowie um eine schnelle und hohe Bioverfügbarkeit im Magen-Darmtrakt sicherzustellen.

Solche Zusätze sind gerade für die orale Applikation an Patienten mit CED als besonders kritisch zu werten, da sie bei nicht erfindungsgemässer Eignung die CED verschlimmern können. So gilt zum Beispiel das bei üblicher Extraktherstellung von anthocyaninhaltigem Extrakt für die Herstellung von Lebensmittelfarben zugesetzte Sulfit als eigentlicher Auslöser von entzündlichen Darmerkrankungen.

Der wie vorstehend beschrieben hergestellte Trockenextrakt wird erfindungsgemäss bevorzugt mit präbiotischen Trägern wie Fruktose-6-Phosphat, Polysacchariden wie Inulin, Lactitol, Stachyose sowie weiterhin Fructanen und Oligofructosen, die dem Fachmann bekannt sind und nicht aufgrund von Flatulenz-Problemen oder irritativen/allergen Eigenschaften vermieden werden müssen, formuliert.

Als weitere Hilfsstoffe kommen erfindungsgemäss bevorzugt je nach Art der gewählten Darreichungsform in Frage: Mikrokristalline Cellulose als Trägerstoff, gesättigte pflanzliche Triglyceride, Gleitmittel wie beispielsweise L-Leucin, Antioxidantien wie beispielsweise Ascorbinsäure, Maltodextrin, Maisstärke, oder Invertzuckersirup.

Erfindungsgemäss kann die vorstehend beschriebene Zubereitung in jeder üblicherweise für die orale Verabreichung eingesetzten Darreichungsform bereitgestellt werden. Es sei diesbezüglich besonders bevorzugt auf Tabletten, Hart- oder Weichkapseln, Lösungen wie Saft oder Sirup, oder mit der erfindungsgemässen Zubereitung versetzte, beschichtete oder überzogene feste Darreichungsformen wie beispielsweise Oblaten oder Fruchtschnitten verwiesen.

Derartige Darreichungsformen und ihre Herstellung sind hinlänglich bekannt und müssen hier nicht näher erläutert werden.

In einer weiteren bevorzugten Ausführungsform der vorliegenden Ausführungsform sind die Bestandteile des Extraktes oder die gesamte Darreichungsform (Tablette) magensaftunlöslich oder retardiert formuliert. Die Herstellung einer derartigen Tablette (beispielsweise durch Beschichten (coating) mit einer magensaftresistenten Schicht) ist dem Fachmann hinlänglich bekannt.

Gemäss einer bevorzugten Ausführungsform der vorliegenden Erfindung wird die erfindungsgemässe Zubereitung einmal täglich in einer Menge von 10 bis 500 mg/kg, vorzugsweise 50 bis 300 mg/kg, besonders bevorzugt 100 bis 200 mg/kg, bezogen auf das Körpergewicht des Patienten verabreicht.

Gemäss der vorliegenden Erfindung kann die erfindungsgemässe Zubereitung auch in Kombination mit anderen Mitteln eingenommen werden, welche einen positiven Einfluss auf *"chronisch-entzündliche Darmerkrankungen"* (CED) haben. Die vorliegende Erfindung betrifft somit auch ein Kombinationspräparat, enthaltend eine erfindungsgemässe Zubereitung und mindestens ein weiteres Präparat, welches einen positiven Einfluss auf *"chronisch-entzündliche Darmerkrankungen"* (CED) hat.

Beispielhaft seien Präparate mit Bakterienstämmen als Inhaltsstoff, oder an Alpha-Säuren reiche Extrakte von Hopfen (Humulus Lupulus) genannt. Derartige Präparate sind im Handel erhältlich. In der EP-1 718 312 B1 ist ein Hopfenextrakt mit hohem Anteil an Alphasäuren für entzündliche Darmerkrankungen beschrieben.

Unter einem Kombinationspräparat wird erfindungsgemäss eine Darreichungsform verstanden, bei welcher die verschiedenen Präparate für eine gemeinsame Verabreichung vorgesehen sind. Es kann sich hierbei um eine feste Dosisform handeln, in welcher die Wirkstoffe in festem Dosisverhältnis in einer einzigen Darreichungsform bereitgestellt sind (beispielsweise einer Tablette). Es kann sich aber auch um eine Darreichungsform handeln, bei welcher die Wirkstoffe getrennt voneinander (z.B. in unterschiedlichen Tabletten oder Kapseln), aber in der gleichen Packung, bereitgestellt werden.

Die erfindungsgemässe Zubereitung ist zur Behandlung und/oder Prophylaxe von *chronisch-entzündlichen Darmerkrankungen"* (CED) vorgesehen. Darunter werden Krankheitsbilder zusammengefasst, die sich durch schubweise rezidivierende oder kontinuierlich auftretende, entzündliche Veränderungen des Darms auszeichnen. Die wichtigsten chronisch-entzündlichen Darmerkrankungen sind Morbus Crohn (MC) und Colitis Ulcerosa (CU). Darüber hinaus beinhaltet der Begriff CED seltenere entzündliche Darmerkrankungen wie die Collagene Colitis (CC). Als Vorstufe zu den CED kann das sogenannte Reizdarmsyndrom (irritable bowel syndrome (IBS) oder auch Colon Irritabile (Reizkolon) genannt) angesehen werden.

Die vorliegende Erfindung wird nachstehend unter Bezugnahme auf nicht einschränkende Zeichnungen und Beispiele näher erläutert.

Es zeigen:
- Fig. 1: die Frucht von zea mays (Mais morado) mit der innenliegenden Spindel
- Fig. 2: eine graphische Darstellung der Entwicklung des DAI-Index in Mäusen mit DSS-induzierter Colitis mit und ohne Gabe der erfindungsgemässen Zubereitung
- Fig. 3: eine graphische Darstellung der Darmdurchlässigkeit von Mäusen mit DSS-induzierter Colitis mit und ohne Gabe der erfindungsgemässen Zubereitung
- Fig. 4: eine graphische Darstellung des Verhältnisses von Gewicht zu Länge des Dickdarms von Mäusen mit DSS-induzierter Colitis mit und ohne Gabe der erfindungsgemässen Zubereitung
- Fig. 5: eine graphische Darstellung der Länge des Dickdarms von Mäusen mit DSS-induzierter Colitis mit und ohne Gabe der erfindungsgemässen Zubereitung

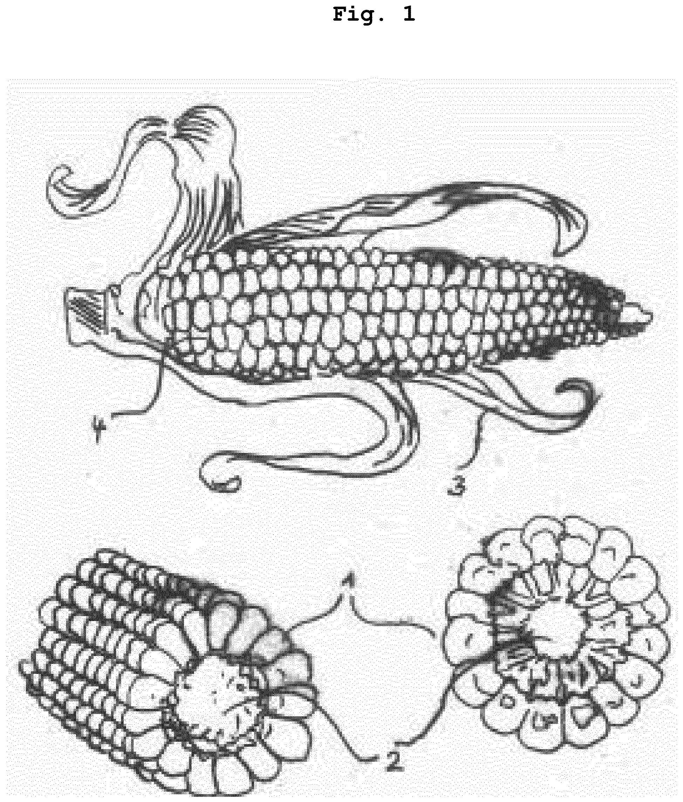

In Fig. 1 ist ein Maiskolben 4 gezeigt, in dessen Innerem sich eine Maisspindel 2 befindet. Der Kolben 4 weist zusätzlich äussere Hüllblätter 3 sowie Körner 1 auf.

### Beispiel 1: Herstellung eines erfindungsgemässen Trockenextrakts

Die Spindeln von Purple Corn (in getrocknetem Zustand), gebrochen zu einer Partikelgrösse von 1-3 mm mittels einer Hammermühle, wurden einer Mazeration/Perkolation unterworfen. Als Extraktionsmittel wurde Ethanol:Wasser 20:80 unter Zusatz von 0.1% HCl gewählt. Die Extraktion wurde anschliessend in einem Soxhlet-Extraktor während 10h bei 50°C (Temperatur des Extraktionsmittels) geführt. Während der Extraktion stellte sich ein pH von 2-3 ein. Die rote flüssige Phase wurde nach Beendigung der Extraktion abgenommen, filtriert und im Vakuum bei <50°C zum Rückstand eingedampft. In der letzten Phase der Trocknung wurde 10% MCC (mikrokristalline Cellulose) als Träger, Fliessverbesserer/ Anticaking-Mittel zugesetzt. Der Trockenextrakt wurde mittels einer Hammermühle auf ca. 100-200 µm eingemahlen.

Der Extrakt wurde mittels HPLC/MS/MS analysiert. Es konnten über 100 einzelne Substanzen identifiziert werden, wovon die Anthocyanin-Fraktion 72% der Peakflächen ausmachte. Polyphenole waren in einer Menge von 5% vertreten. 16% des Extrakts bestanden aus Hydroxy-aromatischen Verbindungen, und 7% des Extrakts aus anderen Komponenten.

Innerhalb der Anthocyanine war das Grundgerüst des Anthocyanidins Cyanidin in rund 75% der Peakflächen vertreten, während Delphinidine nur zu etwa 2% vorhanden waren. Die Derivatisierung war mehrheitlich monoglycosidisch (glycosid, galactosid, 64%) oder acyl-glycosidisch (malonyl-glycosid, 27%).

Dabei war Cyanidin-glucosid mit einem Anteil von 20.86% relativ am häufigsten vertreten (Tabelle 1).

**Tabelle 1: Mengen-Reihenfolge der Anthocyanine im Extrakt**

| % der Anthocyanine | |
|---|---|
| 20.86 | Cyanidin-glucosid |
| 12.30 | Peonidin-glucosid |
| 8.63 | Cyanidin-malonylgalactosid |
| 5.81 | Pelargonidin-glucosid |
| 4.79 | Peonidin-malonylglucosid |
| 3.59 | Cyanidin-galactosid |
| 2.83 | Delphinidin-glucosid |
| 2.00 | Pelargonidin-malonylglucosid |

### Beispiel 2: Herstellung einer Tablette

Eine nachstehend beschriebene Tablettiermischung wurde auf dem Fachmann bekannte Weise durch Kompaktierung, Tablettierung und Coaten in eine Tablette überführt. Die gecoatete Tablette zeigte einen Zerfall in 0.1 N HCL/37°C von >60 min.

| | |
|---|---|
| Purple Corn-Extrakt aus Beispiel 1 | 300 mg |
| MCC (mikrokristalline Cellulose) | 30 mg |
| Inulin | 50 mg |
| Hard fat (Gesättigte pflanzliche Triglyceride) (Baumwollsamenöl) | 14 mg |
| L-Leucine (Gleitmittel) | 6 mg |
| Zein (Corn protein, Magensaftresistenz) (alkoholischer Überzug) | 10 mg |
| Tablette Totalgewicht | 410 mg |
| (rund, 12 mm Durchmesser) | |

### Beispiel 3: Herstellung einer Hartkapsel

Eine übliche Hartkapsel wurde mit folgenden Komponenten befüllt:

| | |
|---|---|
| Purple Corn-Extrakt aus Beispiel 1 | 250mg |
| Ascorbinsäure | 10 mg |
| MCC | 30 mg |
| Inulin | 50 mg |
| Zein (alkohol.) Q.s. | |

Die Komponenten wurden intensiv vorgemischt, im Mischer mit Zein-Lösung (Corn protein, Magensaftresistenz) als alkoholischer Überzug (20 mg/240 mg Granulat) feucht granuliert, und das Granulat nach Passage durch 1 mm Sieb auf Horden bei max. 50°C getrocknet.

Das Granulat (260 mg) wurde in handelsübliche Hartkapseln gefüllt.

### Beispiel 4: Herstellung eines Dicksaftes (Sirups)

Der gemäss Beispiel 1 erhaltene Purple Corn-Extrakt wurde bei der Einengung (siehe Beispiel 1) bei einem Wasser (+Ethanol)-Gehalt von 50% w/w mit der gleichen Menge "Corn Syrup" oder Invertzuckersirup (72-73% Trockensubstanz) versetzt.

Nach Filtration und Stabilisierung mit Ascorbinsäure konnte der Dicksaft direkt heiss abgefüllt (sterilisiert) und in Flaschen zur Zubereitung von heissen oder kalten Getränken oder konzentriert an Patienten dargereicht werden.

### Beispiel 5: Herstellung von Mini-Fruchtschnitten

Folgende Inhaltsstoffe wurden gemischt und über ein Walzwerk zu 5 mm dicken Filmen gewalzt, welche auf Oblaten (Eiweiss, Stärke) von 3- 5 mm Dicke aufgetragen und zu rechteckigen Stängeln (von 4.5 g Zubereitung und 1.5 g Oblate= 6 g) geschnitten wurden.

| | |
|---|---|
| Heidelbeeren getrocknet und gemahlen | 3000 mg |
| Purple Corn Extrakt aus Beispiel 1 | 300 mg |
| Inulin | 300 mg |
| Maltodextrin | 300 mg |
| Maisstärke | 300 mg |
| Invertzuckersirup | 200 mg |

Je nach Verarbeitung kann die Schnitte bei 30°C/20% relativer Luftfeuchte nachgetrocknet werden. Die Schnitten können als Lebensmittel (functional food) eingenommen werden.

Anstelle von Heidelbeeren können weitere oder andere Anthocyaninhaltige Früchte wie Holunderbeeren, Brombeeren, Himbeeren etc. eingesetzt oder zugemischt werden.

### Beispiel 6: Getränk mit Hopfen

Fermentiertem Maisbier (100 Teile geröstete Maiskörner wurden gemahlen, in wässriger Suspension mit Amylase aufgeschlossen und mittels Hefe vergoren) wurde mit Purple Corn-Extrakt aus Beispiel 1 (0.1 Teile) und Hopfenextrakt(0.1 Teile) versetzt, wobei der Hopfenextrakt mittels wässrig-alkoholischer oder CO₂-Extraktion von weiblichen Hopfenblüten erhalten wurde und bis zu 40 % Alphasäuren, insbesondere reduzierte Isoalphasäuren, bevorzugt 20-30% Alphasäuren enthielt. Ein solcher Hopfenextrakt ist zum Beispiel von der Barth-Haas Group zur Bierherstellung erhältlich.

Das so erhaltene Getränk konnte direkt eingenommen werden.

Anstelle von vergorenem Chicha (Maisbier) sind auch alkoholfreies Maisbiere, oder Malzbier verwendbar.

### Beispiel 7: Untersuchung der Wirkung an Mäusen

### Mausmodell

Die Wirkung der erfindungsgemässen Zubereitung wurde mittels des Modells der DSS (dextrane sodium sulphate)-induzierten Colitis in Mäusen überprüft. Dieses Verfahren ist weit verbreitet und wird häufig verwendet. Es ist charakterisiert durch die Verursachung von direkten Schäden an Mucosa/Submucosa im distalen Colon und ist deshalb geeignet um anti-entzündliche Wirkungen von Teststoffen auf menschliche entzündliche Darmkrankheiten (IBD) (Colitis, Morbus Crohn) zu evaluieren. Es hat auch biochemische Verwandtschaft zu menschlicher IBD, insbesondere betreffend Mediatoren wie Eicosanoiden, reaktiven Sauerstoff- und Stickstoff-Spezies sowie Cytokinen.

Männliche C57BL/6J Mäuse (7-9 Wochen alt; 20-25 g) wurden von Charles River (France) beschafft und in Makrolon-Käfigen gehalten (4-5 Mäuse pro Käfig). Die Mäuse wurden in klimatisierter Atmosphäre bei einem 12h-Licht-Dunkel-Zyklus gehalten und hatten freien Zugang zu Leitungswasser und Nahrung. Die Mäuse wurden zufällig in verschiedene Gruppen zu je 9-12 Tieren eingeteilt; zwei dieser Gruppen (nicht-colitische Gruppe und Kontrollgruppe) erhielten das Vehikel, das zur Verabreichung der Testverbindung (Extrakt aus Beispiel 1) verwendet wurde, während die anderen Gruppen den Textextrakt aus Beispiel 1 oral in verschiedenen Mengen (10, 25, 50, 100 and 200 mg/kg) verabreicht bekamen. DSS-Colitis wurde durch Zugabe von DSS (36-50 KDa, MP Biomedicals, Ontario, USA) in einer Konzentration von 3% über einen Zeitraum von 5 Tagen zum Trinkwasser ausgelöst. Nach diesem Zeitraum erhielten die Mäuse normales Leitungswasser.

### DAI-Index

Zur Evaluierung des anti-Entzündungseffekts der Testzubereitung in diesem Modell wurden die verschiedenen Dosen des Extrakts gemäss Beispiel 1 oral verabreicht, beginnend am gleichen Tag wie die Induktion der Colitis und täglich fortgesetzt bis 7 Tage nach dem Beginn des Experiments.

Wasser- und Nahrungsaufnahme wurden während des gesamten Experiments täglich aufgezeichnet. Das Körpergewicht der Tiere, die Anwesenheit von Blut in den Fäkalien, sowie die Stuhlbeschaffenheit wurden täglich für jede Maus von einer Person überprüft, welche keine Kenntnis von der Behandlung hatte. Diese Parameter wurden bewertet, und hieraus wurde ein durchschnittlicher täglicher Krankheitsindex (DAI) berechnet (Tabelle 2).

**Tabelle 2: DAI-Index zur Bewertung der DSS-induzierten Colitis**

| Wert | Gewichtsverlust | Stuhlbeschaffenheit | Rektale Blutung |
|---|---|---|---|
| 0 | Keiner | Normal | Normal |
| 1 | 1 - 5 % | | |
| 2 | 5- 10 % | Lockerer Stuhl | |
| 3 | 10 - 20 % | | |
| 4 | > 20 % | Durchfall | Starke Blutung |

Der DAI-Index wurde als Gesamtwert aus der Summe aus Gewichtsverlust + Stuhlbeschaffenheit + Rektale Blutung, geteilt durch 3, berechnet.

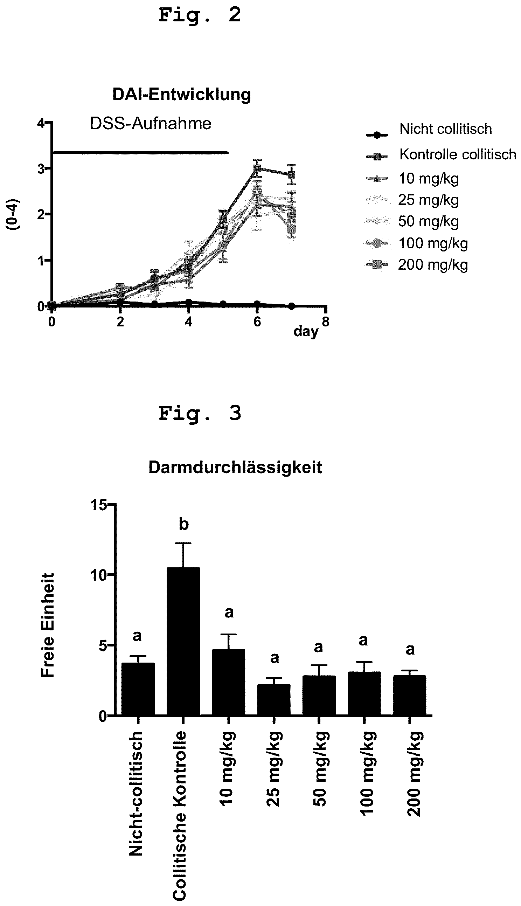

Es zeigte sich, dass der Extrakt gemäss Beispiel 1 eine signifikante Verringerung der Verschlechterung des DAI-Indexes bei Mäusen mit DSS-induzierter Colitis bewirkte. Dies ist in der graphischen Darstellung in Fig. 2 gezeigt.

### Darmdurchlässigkeit (Permeabilität)

Ein in vivo Darmdurchlässigkeitsassay wurde am Tag der Beendigung des Experiments durchgeführt. Mäusen aus den verschiedenen Testgruppen (n=5) wurde nach 12-stündigem Fasten DX-4000-FITC per Sonde zugeführt (Fluorescein-isothiocyanat-carboxymethyl-Dextran, 350 mg/kg Körpergewicht). Nach 4 h wurde durch Herzpunktur Blut entnommen, und bei 4°C und 3000 U/min für 10 min zentrifugiert. Plasma wurde in PBS (pH 7.4) verdünnt (1:50) und mit einem Fluoreszenz-Spektrophotometer (Fluorostart, BMG Labtechnologies, Offenburg, Germany) bei einer Anregungswellenlänge von 485 nm und einer Emissionswellenlänge von 535 nm auf die DX-4000-FITC-Konzentration untersucht. Standardkurven wurden durch Verdünnung von FITC-Dextran in PBS (Phosphat-gepufferte Kochsalzlösung) erhalten.

Es wurde gefunden, dass alle untersuchten Dosen des Extrakts gemäss Beispiel 1 die Darmfunktionalität durch Verbesserung der Darmdurchlässigkeit erhöhten. Die FITC-Dextran-Plasmaspiegel waren in den colitischen Kontrollmäusen signifikant höher als in den nicht-colitischen Mäusen, was der durch DSS induzierten Veränderung der epithelen Barrierefunktion entspricht. Die mit dem Extrakt gemäss Beispiel 1 behandelten Mäuse wiesen jedoch signifikant verringerte FITC-Dextran-Plasmaspiegel auf (Figur 3; P<0.05; Ergebnisse mit unterschiedlichem Buchstaben (z.B. a,b) unterscheiden sich statistisch signifikant).

### Darmlänge und -gewicht

Nach Beendigung des Experiments wurde den geopferten Mäusen der Dickdarm entnommen und auf eine eiskalte Platte gegeben. Die Darmbestandteile wurden gesammelt, von Fett und Gekröse gereinigt und danach auf Filterpapier aufgetragen. Jede Probe wurde gewogen und bei einer konstanten Last (2 g) einer Längenbestimmung unterzogen.

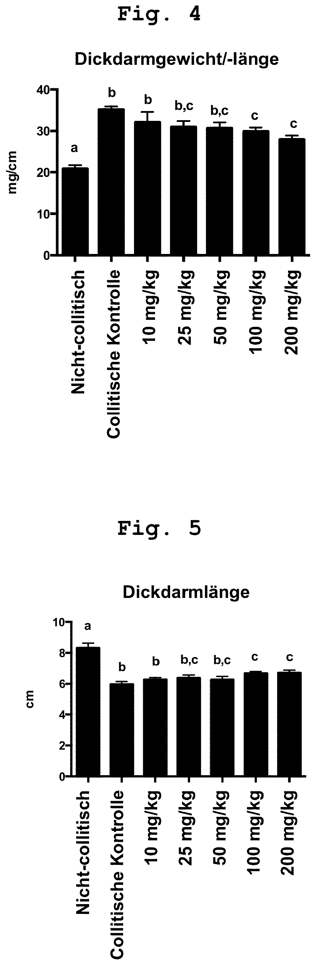

Es zeigte sich, dass das Gewicht/Länge-Verhältnis bei colitischen Mäusen signifikant erhöht war, im Vergleich zur nicht-colitischen Gruppe (Figur 4; Ergebnisse mit unterschiedlichem Buchstaben (z.B. a,b) unterscheiden sich statistisch signifikant). Dies war kennzeichnend für die Gewebeödeme, welche bei durch DSS induzierter Dickdarmentzündung auftreten und mit einer Verkürzung der Dickdarmlänge verbunden sind. Die Gruppen colitischer Mäuse, welche 100 oder 200 mg/kg des Extrakts von Beispiel 1 erhielten, zeigten im Vergleich zur colitischen Kontrollgruppe ohne Behandlung eine statistisch signifikante Verbesserung (Figur 4).

Dieser Effekt ging einher mit einer Verbesserung der Länge der Darmproben, welche bei den Mäusen, welche 100 oder 200 mg/kg des Extrakts aus Beispiel 1 erhielten, signifikant erhöht war (Figur 5; Ergebnisse mit unterschiedlichem Buchstaben (z.B. a,b) unterscheiden sich statistisch signifikant).

## Patentansprüche

1. Zubereitung, enthaltend einen Anthocyanin-haltigen Extrakt aus den Spindeln und/oder den äusseren Hüllblättern von blauem Mais, vorzugsweise Mais Morado, zur oralen Behandlung von und/oder Vorbeugung gegen chronisch-entzündliche Darmerkrankungen beim Menschen, wobei die chronisch-entzündliche Darmerkrankung vorzugsweise ausgewählt ist aus der Gruppe bestehend aus Colitis, insbesondere von Colitis Irritabile, Colitis Ulcerosa, Morbus Crohn, und Reizdarm-Syndrom.

2. Zubereitung nach Anspruch 1, **dadurch gekennzeichnet, dass** die Zubereitung einen Anteil von Anthocyaninen von mindestens 10 Gew.-%, vorzugsweise im Bereich von 50 bis 80 Gew.-%, bevorzugter 60 bis 75 Gew.-% und besonders bevorzugt 65 bis 75 Gew.-%, bezogen auf die Gesamttrockenmenge des Extrakts aufweist.

3. Zubereitung nach Anspruch 1 oder 2, **dadurch gekennzeichnet, dass** die Zubereitung einen Anteil an Anthocyaninen in Form von Monoglycosiden aufweist, der im Bereich von 30 bis 90 Gew.-%, bevorzugt 50 bis 80 Gew.-%, vorzugsweise 60 bis 75 Gew.-% und besonders bevorzugt 60 bis 70 Gew.-% liegt, bezogen auf die Gesamttrockenmenge der enthaltenen Anthocyanine.

4. Zubereitung nach einem der Ansprüche 1 bis 3, **dadurch gekennzeichnet, dass** die Zubereitung einen Anteil an Polyphenolen aufweist, der im Bereich von 1 bis 10 Gew.-%, vorzugsweise 2 bis 8 Gew.-% und besonders bevorzugt 3 bis 6 Gew.-% liegt, bezogen auf die Gesamttrockenmenge des Extrakts.

5. Zubereitung nach einem der Ansprüche 1 bis 4, **dadurch gekennzeichnet, dass** die Zubereitung zusätzlich Hilfsstoffe enthält.

6. Zubereitung nach Anspruch 5, **dadurch gekennzeichnet, dass** als ein Hilfsstoff eine Substanz, ausgewählt aus präbiotischen Trägern wie Fruktose-6-Phosphat, Polysacchariden wie Inulin, Lactitol, Stachyose sowie weiterhin Fructanen und Oligofructosen, enthalten ist.

7. Verfahren zur Herstellung einer Zubereitung gemäss einem der Ansprüche 1 bis 6, umfassend den Schritt der Behandlung der Spindel und/oder der äusseren Hüllblättern von blauem Mais, vorzugsweise Mais Morado, mit einem wässrigen Medium, vorzugsweise einem wässrig-alkoholischen Medium, besonders bevorzugt einem Wasser-Ethanol-Gemisch, wobei das wässrige Medium einen pH-Wert von 2 bis 5, bevorzugt, 2 bis 3 aufweist.

8. Verfahren nach Anspruch 7, **dadurch gekennzeichnet, dass** die Behandlung unter Zusatz von Ascorbinsäure und/oder Betain durchgeführt wird.

9. Verfahren nach Anspruch 7 oder 8, **dadurch gekennzeichnet, dass** die Spindel und/oder der äusseren Hüllblättern von blauem Mais, vorzugsweise Mais Morado, vor der Behandlung mit wässrigem Medium zerkleinert werden, vorzugsweise zu Partikeln mit einer durchschnittlichen Partikelgrösse von 1- 10 mm, bevorzugt 1-5 mm.

10. Verfahren nach einem der Ansprüche 7 bis 9, **dadurch gekennzeichnet, dass** ein Wasser-Ethanol-Gemisch mit einem Verhältnis von Wasser:Ethanol von 90:10 bis 60:40, insbesondere bevorzugt 85:30 und speziell 80:20, als wässriges Medium eingesetzt wird.

11. Verfahren nach einem der Ansprüche 7 bis 10, **dadurch gekennzeichnet, dass** die Behandlung bei einer Temperatur von 30 bis 90°C, vorzugsweise 40 bis 70°C, über einen Zeitraum von 1 bis 24 h, beispielsweise 5 bis 15 h durchgeführt wird.

12. Verfahren nach einem der Ansprüche 5 bis 9, **dadurch gekennzeichnet, dass** nach dem Schritt der Behandlung ein Trocken-Extrakt hergestellt wird.

13. Kombinationspräparat, enthaltend eine Zubereitung gemäss einem der Ansprüche 1 bis 6 und mindestens ein weiteres Präparat, welches einen positiven Einfluss auf *"chronisch-entzündliche Darmerkrankungen"* (CED) hat.

14. Kombinationspräparat nach Anspruch 13, **dadurch gekennzeichnet, dass** das weitere Präparat ausgewählt ist aus der Gruppe bestehend aus Präparaten mit Bakterienstämmen als Inhaltsstoff, oder an Alpha-Säuren reiche Extrakten von Hopfen.

15. Verwendung einer Zubereitung gemäss einem der Ansprüche 1 bis 6 beziehungsweise eines Kombinationspräparats gemäss einem der Ansprüche 13 bis 14 zur Behandlung und/oder Prophylaxe von chronisch-entzündlichen Darmerkrankungen, vorzugsweise ausgewählt aus der Gruppe bestehend aus Morbus Crohn, Colitis, insbesondere Colitis Irritabile oder Colitis Ulcerosa, Collagene Colitis, lymphozytäre Colitis und Reizdarm-Syndrom.
